# EUROPÄISCHE PATENTSCHRIFT

(11) **EP 2 941 283 B1**
(45) Veröffentlichungstag und Bekanntmachung des Hinweises auf die Patenterteilung: **05.04.2017**
(21) Anmeldenummer: 14702194.3
(22) Anmeldetag: 06.01.2014
(51) Int. Cl.: A61M 1/16, A61M 1/36

(54) **VORRICHTUNG UND VERFAHREN ZUR ENTFERNUNG PROTEINGEBUNDENER TOXINE AUS DEM BLUT VON PATIENTEN UNTER EINSATZ EINES HOCHFREQUENTEN, ELEKTROMAGNETISCHEN FELDES UND EINES ELEKTROSTATISCHEN GLEICHFELDES**
DEVICE AND METHOD FOR REMOVING PROTEIN-BOUND TOXINS FROM THE BLOOD OF PATIENTS USING A HIGH-FREQUENCY, ELECTROMAGNETIC FIELD AND AN ELECTROSTATIC DIRECT CURRENT FIELD
DISPOSITIF ET PROCÉDÉ D'ÉLIMINATION DE TOXINES LIÉES À DES PROTÉINES PROVENANT DU SANG DE PATIENTS EN UTILISANT UN CHAMP ÉLECTROMAGNÉTIQUE À HAUTE FRÉQUENCE ET UN CHAMP ÉLECTROSTATIQUE CONTINU

(30) Priorität: 04.01.2013 DE 102013100050
(43) Veröffentlichungstag der Anmeldung: 11.11.2015
(73) Patentinhaber: Fresenius Medical Care Deutschland GmbH, 61352 Bad Homburg (DE)
(72) Erfinder: JANKOWSKI, Joachim, 52159 Roetgen (DE); FABIG, Anselm, 15738 Zeuthen (DE); TSCHULENA, Ulrich, 60439 Frankfurt (DE); MÜLLER, Carsten, 97502 Euerbach (DE)
(74) Vertreter: Gulde & Partner
(86) Internationale Anmeldenummer: PCT/EP2014/050080
(87) Internationale Veröffentlichungsnummer: WO 2014/106654

(56) Entgegenhaltungen:
- WO-A1-2014/095072
- WO-A1-2014/095073
- RO-B1- 122 077
- US-A1- 2003 187 380
- US-A1- 2005 015 040
- US-A1- 2005 082 225

## Beschreibung

Primäre Aufgabe der Nieren ist die Ausscheidung harnpflichtiger Substanzen, den sogenannten Urämie-Toxinen. Nieren chronisch niereninsuffizienter Patienten können diese Aufgaben nicht mehr erfüllen, was im unbehandelten Zustand rasch zur Vergiftung des Patienten und damit zum Tod führt. Die Dialyse ist das Instrument der Wahl zur Linderung der akuten und chronischen Erkrankung, und zur zeitlichen Überbrückung bis ein geeignetes Spenderorgan zur Verfügung steht. Die Dialyse beruht auf dem Prinzip des Stoffaustauschs durch Filtration bzw. Diffusion. Dabei wirken die derzeit verwendeten Membranen als reine Filter- und/oder Diffusionsmembranen und sorgen dafür, dass dem zu reinigenden Blut Stoffe entzogen werden, die eine bestimmte Größe nicht überschreiten. Durch die derzeit angewandten Dialysetechniken ist aber im Regelfall eine vollständige Abtrennung der Urämie-Toxine nicht möglich, da ein Teil der harnpflichtigen Substanzen proteingebunden vorliegen. Hierbei handelt sich unter anderem um niedermolekulare, aromatische Substanzen. Urämie-Toxine, die in der Regel proteingebunden vorliegen, sind beispielsweise Phenylessigsäure, p-Hydroxyhippursäure und Indoxylsulfat. In der Folge akkumulieren die betreffenden Substanzen im Organismus des Patienten und bedingen dort Folgeerkrankungen der akuten und chronischen Niereninsuffizienz. Bei chronisch niereninsuffizienten Patienten treten dadurch bedingt zunehmend Folgeerkrankungen wie Herzkreislauferkrankungen, und daraus resultierend eine erhöhte Sterblichkeitsrate auf.

Aromatische, hydrophobe Urämie-Toxine weisen eine geringe Wasserlöslichkeit auf. Daher kommt es zwischen diesen Substanzen und Plasmaproteinen meist zur Ausbildung adsorptiver Effekte. Diese adsorptiven Effekte gehen auf unterschiedliche Wechselwirkungen zurück. Hierbei sind vor allem Wasserstoffbrückenbindungen, lonenbindungen und Dipol-Dipol-Wechselwirkungen (van-der-Waals-Kräfte) zu nennen. Durch die Bindung an Plasmaproteine, wie z. B. Albumin, kann das effektive Molekulargewicht der harnpflichtigen Substanzen erhöht werden. Das resultierende Molekulargewicht der proteingebundenen Urämie-Toxine liegt dadurch meist oberhalb der Ausschlussgrenze der verwendeten Dialysemembranen und diese können somit bei der Dialyse nicht effektiv entfernt werden.

Durch die Dialyse kann folglich nur der nicht-proteingebundene Anteil des betreffenden Urämie-Toxins abgetrennt werden. Der proteingebundene Anteil (bis zu 95 % der Gesamtmenge des Urämie-Toxins) wird dadurch nicht grundlegend verändert. Aufgrund des Gleichgewichts gemäß des Massenwirkungsgesetz zwischen proteingebundenen und nicht-proteingebundenen Urämie-Toxinen werden unmittelbar nach der Dialyse wieder im Wesentlichen die initialen, nicht-proteingebundene Urämie-Toxinkonzentrationen im Plasma chronischniereninsuffizienter Patienten erreicht. Da die pathophysiologischen und pathochemischen Wirkungen im Besonderen von nicht-proteingebundenen Urämie-Toxinen bedingt werden, wird durch diese Gleichgewichtseinstellung ein für die Patienten fataler Prozess initiiert. Dieser *circulus vitiosus* ist Ursache für die vielfältigen pathologischen Erscheinungsformen der chronischen Niereninsuffizienz. Bis zum heutigen Tag sind keine konventionellen Methoden verfügbar, durch die auch protein-gebundene Urämie-Toxine während der Dialyse effektiv aus dem zu reinigenden Blut entfernt werden können.

In WO 2014/095072 A1 sowie WO 2014/095073 A1 sind beispielhafte Ausführungsformen von Dialysegeräten des Standes der Technik offenbart.

Der vorliegenden Erfindung liegt die Aufgabe zugrunde mindestens einen Nachteil des geschilderten Standes der Technik zu vermindern oder zu vermeiden. Insbesondere ist es eine Aufgabe der Erfindung Mittel und Wege zur Verfügung zu stellen, mit denen proteingebundene Urämie-Toxine effektiv aus dem Blut von Dialysepatienten entfernt werden können.

Die Aufgabe wird gelöst durch die Bereitstellung eines Dialysegerätes nach einem der Ansprüche 1 bis 13. Das erfindungsgemäße Dialysegerät beruht auf der Verwendung eines hochfrequenten, elektromagnetischen Feldes und eines elektrostatischen Gleichfeldes in einem Verfahren zur Dialyse mittels eines Dialysators zum Stoffaustausch, insbesondere zur Hämodialyse oder Hämodiafiltration.

Der Erfindung liegt die Erkenntnis zugrunde, dass die Bindungen zwischen Urämie-Toxinen und Plasmaproteinen in der Regel keine *"echten"* chemischen (kovalenten) Bindungen sind, sondern es sich um reversible Bindungen handelt. Diese Bindungen beruhen im Wesentlichen auf den elektrostatischen Eigenschaften und Wechselwirkungen der beteiligten Moleküle. Es wurde gefunden, dass die Stärke dieser Bindungen oder Wechselwirkungen durch die Einwirkung von hochfrequenten elektromagnetischen Feldern herabgesetzt werden kann.

Durch die Verwendung hochfrequenter elektromagnetischer Felder während der Dialyse kann der Anteil proteingebundener Urämie-Toxine deutlich reduziert werden. Im Rahmen der im klinischen Alltag durchgeführten Dialyse kann durch zusätzliche Anwendung hochfrequenter elektromagnetischer Felder die Freisetzungsrate protein-gebundener Urämie-Toxine aus der Proteinbindung erhöht werden. Die betreffenden Urämie-Toxine können somit vermehrt und effektiver dialysiert werden. Die aus der Proteinbindung freigesetzten Urämie-Toxine tragen mehrheitlich eine positive oder negative elektrische Ladung. Durch das Einbringen eines statischen oder rotierenden elektrostatischen Gleichfeldes können die aus der Proteinbindung freigesetzten Urämie-Toxine im Dialysator in Richtung der Dialysemembran bewegt werden. Damit wird eine Zunahme des Diffusionsdrucks bzgl. dieser Urämie-Toxine während der Dialyse erreicht und die aus der Proteinbindung freigesetzten Urämie-Toxine werden effektiver aus dem zu reinigenden Blut entfernt. In der erfindungsgemäßen Lösung wird also zunächst durch das Einwirken eines hochfrequenten elektromagnetischen Feldes vermehrt Urämie-Toxin aus seiner Proteinbindung freigesetzt und durch das Einwirken eines elektrostatischen Gleichfeldes wird das zuvor freigesetzte Urämie-Toxin gerichtet Richtung Dialysemembran bewegt und somit vermehrt aus dem zu reinigenden Blut entfernt. Im Ergebnis wird eine verbesserte Entfernung von Proteingebundenen Urämie-Toxinen aus dem zu reinigenden Blut erreicht.

Die vorliegende Erfindung bezieht sich insbesondere auf ein Dialysegerät. Ein Dialysegerät umfasst in der Regel einen Dialysatkreislauf, einen Blutkreislauf und einen Dialysator für den Stoffaustausch zwischen zu reinigendem Blut des Blutkreislaufs und Dialysat des Dialysatkreislaufs. Das erfindungsgemäße Dialysegerät zeichnet sich dadurch aus, dass es Mittel aufweist zur Erzeugung eines hochfrequenten elektromagnetischen Feldes sowie Mittel zur Erzeugung eines elektrostatischen Gleichfeldes und wobei beide Mittel derart ausgeführt und angeordnet sind, dass zu reinigendes Blut während der Passage durch den Dialysator dem hochfrequenten elektromagnetischen Feld und dem elektrostatischen Gleichfeld ausgesetzt werden können.

Das erfindungsgemäße Dialysegerät weist einen Dialysatkreislauf auf. Im Dialysatkreislauf wird das Dialysat bewegt, gegen welches das zu reinigende Blut dialysiert werden soll. Dabei wird unter dem Begriff "Dialysatkreislauf" eine Leitungsanordnung verstanden, in der das Dialysat aus einem Reservoir z. B. durch eine Pumpe derart gerichtet durch den Dialysator bewegt werden kann, dass die Dialysatflüssigkeit den Dialysator im Gegenstrom zum zu reinigenden Blut auf der dem Blut abgewandten Seite der Membran des Dialysators geführt wird. Nach der Passage durch den Dialysator kann das Dialysat abgeführt und ggf. in einem weiteren Behälter aufgefangen und gesammelt werden. Alternativ dazu kann das Dialysat dem Dialysatkreislauf für eine weitere Passage des Dialysators zugeführt werden.

Das erfindungsgemäße Dialysegerät weist einen Blutkreislauf auf. Im Blutkreislauf wird das zu reinigende Blut bewegt. Dabei wird unter dem Begriff "Blutkreislauf" eine Leitungsanordnung verstanden, in der das zu reinigende Blut dem Patienten entnommen wird und z. B. durch eine Pumpe derart gerichtet durch den Dialysator bewegt werden kann, dass das zu reinigende Blut den Dialysator im Gegenstrom zum Dialysat auf der dem Dialysat abgewandten Seite der semipermeablen Membran des Dialysators geführt wird. Nach der Passage durch den Dialysator wird das gereinigte Blut dem Patienten wieder zurückgeführt.

Im erfindungsgemäßen Dialysegerät wird ein Dialysator zum Stoffaustausch verwendet. Aufgabe des Dialysators ist es, aus dem zu reinigenden Blut Urämie-Toxine möglichst effektiv zu entfernen. Im Dialysator sind zu reinigendes Blut und eine Flüssigkeit, gegen die dialysiert werden soll, das sog. Dialysat, durch eine semipermeable Membran voneinander getrennt. In der Regel wird im Dialysator das Dialysat in einem Dialysatkreislauf im Gegenstrom zum Blutfluss im Blutkreislauf geführt. Über die semipermeable Membran des Dialysators erfolgt der Stoffaustausch zwischen zu reinigendem Blut auf der einen Seite und Dialysat auf der anderen Seite der Membran. Dabei erfolgt der Stofftransport der Urämie-Toxine über die Membran durch Diffusion oder Konvektion. Die Selektivität des Stoffaustausches wird einerseits durch die Eigenschaften der Membran, wie z. B. der Porengröße, bestimmt, andererseits durch die Zusammensetzung des Dialysates. Geeignete Dialysatoren sind im Stand der Technik beschrieben und deren Verwendung ist dem Fachmann bekannt. Üblicherweise werden Kapillardialysatoren eingesetzt. Der Dialysator umfasst bevorzugt eine semipermeable Membran mit einer Größenausschlussgrenze die ausgewählt ist aus dem Bereich von 10.000 bis 25.000 Da, bevorzugt von 14.000 bis 17.000 Da.

Das zu reinigende Blut wird während der Passage des zu reinigenden Blutes durch den Dialysator bzw. während eines Kontaktes des zu reinigenden Blutes mit einer semipermeablen Membran des Dialysators einem hochfrequenten elektromagnetischen Feld ausgesetzt. Dazu weist das erfindungsgemäße Dialysegerät Mittel auf zur Erzeugung eines hochfrequenten elektromagnetischen Feldes.

Die Mittel zur Erzeugung eines hochfrequenten, elektromagnetischen Feldes können derart ausgestaltet und angeordnet sein, dass das zu reinigende Blut dem hochfrequenten elektromagnetischen Feld während der Passage des zu reinigenden Blutes durch den Dialysator bzw. während eines Kontaktes mit einer semipermeablen Membran des Dialysators ausgesetzt werden kann. Dazu können die Mittel zur Erzeugung des hochfrequenten, elektromagnetischen Feldes derart angeordnet sein, dass ein Teil, ein überwiegender Teil oder der gesamte Dialysator entlang der Achse der Flussrichtung des zu reinigenden Blutes unmittelbar dem hochfrequenten, elektromagnetischen Feld ausgesetzt werden kann. Insbesondere können die Mittel zur Erzeugung des hochfrequenten, elektromagnetischen Feldes an der Außenfläche des Dialysators des erfindungsgemäßen Dialysegerätes angeordnet sein oder einen integralen Bestandteil des Dialysators bilden.

In einer bevorzugten Variante sind die Mittel zur Erzeugung eines hochfrequenten elektromagnetischen Feldes derart angeordnet, dass das zu reinigende Blut bereits bei Eintritt in den Dialysator dem hochfrequenten elektromagnetischen Feld ausgesetzt wird. Dies hat den Vorteil, dass die Freisetzung der Urämie-Toxine aus der Proteinbindung bereits zu Beginn der Passage durch den Dialysator erfolgt und somit die volle Kapazität des Dialysators für den Stoffaustausch mit dem Dialysat zur Verfügung steht. Wird das zu reinigende Blut während des Kontaktes mit dem Dialysator dem hochfrequenten elektromagnetischen Feld ausgesetzt, so kann das zu reinigende Blut während der gesamten Passage durch den Dialysator dem hochfrequenten elektromagnetischen Feld ausgesetzt sein oder nur für einen Teil der Passage. Es ist auch möglich, dass die Mittel zur Erzeugung eines hochfrequenten elektromagnetischen Feldes derart im Dialysegerät angeordnet sind, dass das zu reinigende Blut an mehreren Stellen der Passage durch den Dialysator einem hochfrequenten elektromagnetischen Feld ausgesetzt wird.

Im erfindungsgemäßen Dialysegerät können Mittel eingesetzt werden, die hochfrequente elektromagnetische Felder mit einer Frequenz von beispielsweise 100 kHz bis 1 GHz erzeugen, bevorzugt von 10 MHz bis 500 MHz, besonders bevorzugt von 80 MHz bis 170 MHz, ganz besonders bevorzugt von 100 MHz bis 120 MHz, weiterhin bevorzugt von 110 MHz bis 115 MHz sowie insbesondere bevorzugt von 110 MHz bis 113 MHz und von 110 MHz bis 111 MHz. Alternative bevorzugte Bereiche für die Frequenz der zu erzeugenden hochfrequenten elektromagnetischen Felder sind 0,5 MHz bis 100 MHz, besonders bevorzugt 1 MHz bis 50 MHz, ganz besonders bevorzugt 1 MHz bis 20 MHz.

Dabei können die Mittel derart ausgestaltet sein, dass das zu reinigende Blut einem hochfrequenten elektromagnetischen Feld ausgesetzt wird, welches im Wesentlichen über die Zeit eine konstante Frequenz aufweist. Alternativ kann das hochfrequente elektromagnetische Feld eine variierende Frequenz aufweisen, wobei die Frequenz und/oder Feldstärke in einem regelmäßigen oder einem unregelmäßigen Muster variiert werden kann. In einer beispielhaften Ausführungsform wird das zu reinigende Blut einem hochfrequenten elektromagnetischen Feld ausgesetzt welches mit einer relativ geringen Frequenz beginnt, beispielsweise 1 MHz, und dessen Frequenz über die Zeit bis zu einer vorher festgelegten maximalen Frequenz gesteigert wird, beispielsweise 20 MHz, oder beispielsweise von 100 MHz bis 170 MHz. Alternativ dazu kann das zu reinigende Blut auch einem hochfrequenten elektromagnetischen Feld ausgesetzt werden, welches mit einer hohen Maximalfrequenz beginnt und dessen Frequenz über die Zeit bis zu einer vorher festgelegten Mindestfrequenz verringert wird. Durch die Verwendung von Mitteln für die Erzeugung von hochfrequenten elektromagnetischen Feldern mit variierenden Frequenzen wird die Effektivität der Freisetzung von Bindungen zwischen Urämie-Toxin und Plasmaprotein verbessert.

Um eine effektive Aufhebung der Bindung zwischen Urämie-Toxin und Plasmaprotein zu erreichen, ist es von Vorteil, das hochfrequente elektromagnetische Feld auf das zu reinigende Blut/Plasma für eine bestimmte Zeitspanne einwirken zu lassen, so dass es zur Schwingungsanregung von Atomen der beteiligten Moleküle bzw. der Moleküle insgesamt kommen kann. Dazu kann das zu reinigende Blut erfindungsgemäß dem hochfrequenten elektromagnetischen Feld mindestens für eine Zeitdauer von 1/10 Sekunden ausgesetzt sein, bevorzugt für eine Zeitdauer von mindestens ½ Sekunden, besonders bevorzugt über eine Zeitdauer von mindestens einer Sekunde.

Um eine möglichst effektive Trennung von Urämie-Toxin und Plasmaprotein zu erreichen kann es von Vorteil sein, im erfindungsgemäßen Dialysegerät Mittel anzuordnen, die hoch elektromagnetische Felder erzeugen mit einer bestimmten elektrischen oder magnetischen Feldstärke. So werden bevorzugt Mittel eingesetzt zur Erzeugung eines hochfrequenten elektromagnetischen Feldes, welches eine elektrische Feldstärke von ≤ 250 V/m, insbesondere von 1 bis 100 V/m, bevorzugt von 1 bis 10 V/m aufweist. Es können beispielsweise Mittel verwendet werden zur Erzeugung eines hochfrequenten elektromagnetischen Feldes, welches eine magnetische Flussdichte aufweist von ≤ 100 mTesla, bevorzugt von 0,001 bis 100 mTesla, besonders bevorzugt von 0,01 bis 10 mTesla, insbesondere von 0,01 bis 2 mTesla. In einer besonderen Ausführungsform werden Mittel verwendet zur Erzeugung eines hochfrequenten elektromagnetischen Feldes, welches eine magnetische Flussdichte aufweist von ca. 31 mTesla.

Dem Fachmann sind Mittel und Verfahren zur Erzeugung geeigneter hochfrequenter, elektromagnetischer Felder bekannt, wie beispielsweise geeignete Feldgeneratoren. Das erfin dungsgemäße Dialysegerät kann zur Erzeugung eines hochfrequenten, elektromagnetischen Feldes beispielsweise eine Hochfrequenzspule, eine Hochfrequenzelektrode und/oder einen Hochfrequenzkondensator aufweisen.

Im erfindungsgemäßen Dialysegerät kann das zu reinigende Blut während der Passage des zu reinigenden Blutes durch den Dialysator bzw. während eines Kontaktes des zu reinigen Blutes mit einer semipermeablen Membran des Dialysators einem hochfrequenten elektromagnetischen Feld und einem elektrostatischen Gleichfeld ausgesetzt werden. Dazu weist das erfindungsgemäße Dialysegerät Mittel auf zur Erzeugung eines elektrostatischen Gleichfeldes.

Ein elektrostatisches Gleichfeld ist ein elektrostatisches Feld, das in seiner Ausrichtung zeitlich konstant ist, im Gegensatz zu einem sich periodisch ändernden Wechselfeld. Elektrostatische Gleichfelder können beispielsweise von elektrischen Leitern oder Feldplatten erzeugt werden, an die ein Gleichstrom angelegt ist.

Die Mittel zur Erzeugung eines elektrostatischen Gleichfeldes können derart ausgestaltet und angeordnet sein, dass das zu reinigende Blut dem elektrostatischen Gleichfeld während der Passage des zu reinigenden Blutes durch den Dialysator bzw. während eines Kontaktes mit einer semipermeablen Membran des Dialysators ausgesetzt werden kann. Dazu können die Mittel zur Erzeugung eines elektrostatischen Gleichfeldes derart angeordnet sein, dass ein Teil, ein überwiegender Teil oder der gesamte Dialysator entlang der Achse der Flussrichtung des zu reinigenden Blutes unmittelbar dem elektrostatischen Gleichfeld ausgesetzt werden kann. Insbesondere können die Mittel zur Erzeugung des elektrostatischen Gleichfeldes an der Außenfläche des Dialysators des erfindungsgemäßen Dialysegerätes angeordnet sein oder einen integralen Bestandteil des Dialysators bilden. Bevorzugt sind die Mittel zur Erzeugung eines hochfrequenten elektromagnetischen Feldes und die Mittel zur Erzeugung eines elektrostatischen Gleichfeldes derart angeordnet, dass sich das hochfrequente, elektromagnetische Feld und das elektrostatische Gleichfeld ganz oder teilweise überlagern. Alternativ können die Mittel zur Erzeugung eines hochfrequenten elektromagnetischen Feldes und die Mittel zur Erzeugung eines elektrostatischen Gleichfeldes derart angeordnet sein, dass das elektrostatische Gleichfeld dem hochfrequenten, elektromagnetischen Feld in Flussrichtung des zu reinigenden Blutes nachgeschaltet ist. Insbesondere können die Mittel zur Erzeugung des elektrostatischen Gleichfeldes derart ausgebildet und angeordnet sein, dass das elektrostatische Gleichfeld im Wesentlichen nicht parallel zur Flussrichtung des zu reinigenden Blutes durch den Dialysator ausgerichtet ist. Bevorzugt sind die Mittel zur Erzeugung des elektrostatischen Gleichfeldes derart im Dialysator angeordnet, dass positiv oder negativ geladene Urämie-Toxine im zu reinigenden Blut durch das elektrostatische Gleichfeld gerichtet auf die Dialysemembran des Dialysators zu bewegt werden.

Die Mittel zur Erzeugung eines elektrostatischen Gleichfeldes können im Dialysegerät derart angeordnet sein, dass das zu reinigende Blut während der gesamten Passage durch den Dialysator dem elektrostatischen Gleichfeld ausgesetzt ist oder nur für einen Teil der Passage. Es ist auch möglich, dass die Mittel zur Erzeugung eines elektrostatischen Gleichfeldes im Dialysegerät derart angeordnet sind, dass das zu reinigende Blut an mehreren Stellen der Passage durch den Dialysator einem elektrostatischen Gleichfeld ausgesetzt wird. In einer bevorzugten Variante sind die Mittel zur Erzeugung eines elektrostatischen Gleichfeldes derart angeordnet, dass das zu reinigende Blut bereits bei Eintritt in den Dialysator und im Wesentlichen über die gesamte Passage durch den Dialysator dem elektrostatischen Gleichfeld ausgesetzt wird. Dies hat den Vorteil, dass die Eliminierung positiv oder negativ geladener, aus der Proteinbindung freigesetzter Urämie-Toxine im Wesentlichen über die gesamte Länge des Dialysators erfolgen kann und somit die volle Kapazität des Dialysators für den Stoffaustausch mit dem Dialysat ausgeschöpft werden kann.

Um eine möglichst effektive Eliminierung von aus der Proteinbindung freigesetzten, geladenen Urämie-Toxinen zu erreichen kann es von Vorteil sein, im erfindungsgemäßen Dialysegerät Mittel anzuordnen, die elektrostatische Gleichfelder erzeugen mit einer bestimmten elektrischen Feldstärke. So werden bevorzugt Mittel eingesetzt zur Erzeugung eines elektrostatischen Gleichfeldes, welches eine elektrische Feldstärke von ≤ 5000 V/m, insbesondere von ≤ 1500 V/m, bevorzugt von 0,1 bis 1500 V/m, besonders bevorzugt von 1 bis 1000 V/m aufweist. In einer besonderen Ausführung des erfindungsgemäßen Dialysegerätes werden Mittel eingesetzt zur Erzeugung eines elektrostatischen Gleichfeldes, welches eine elektrische Feldstärke von ca. 250 V/m aufweist.

Dem Fachmann sind Mittel und Verfahren zur Erzeugung geeigneter elektrostatischer Gleichfelder bekannt, wie beispielsweise geeignete Feldgeneratoren. Das erfindungsgemäße Dialysegerät kann zur Erzeugung eines elektrostatischen Gleichfeldes beispielsweise mindestens zwei elektrische Leiter oder Feldplatten aufweisen zwischen denen das elektrostatische Gleichfeld erzeugt wird, wobei die mindestens zwei elektrischen Leiter oder Feldplatten auf gegenüberliegenden Seiten des Dialysators angeordnet sind. Die Mittel zur Erzeugung eines elektrostatischen Gleichfeldes weisen im erfindungsgemäßen Dialysegerät
mehr als zwei gegenüberliegende elektrische Leiter auf, wobei die elektrischen Leiter derart um den Dialysator angeordnet sind, dass das elektrostatische Gleichfeld um die Achse entlang der Flussrichtung des zu reinigenden Blutes durch den Dialysator rotierbar ist. In diesem Fall kann das elektrostatische Gleichfeld nicht nur statisch betrieben werden, sondern bei Bedarf auch rotierend. Dabei ist die Rotationsgeschwindigkeit langsamer gewählt als die Frequenz des hochfrequenten elektromagnetischen Feldes. Die Rotation des elektrostatischen Gleichfeldes erfolgt bevorzugt mit einer Frequenz von 100 kHz bis 100 MHz, besonders bevorzugt mit einer Frequenz von 0,5 MHz bis 50 MHz, ganz besonders bevorzugt von 1 MHz bis 25 MHz sowie insbesondere bevorzugt von 1 MHz bis 6 MHz und/oder 9 MHz bis 13 MHz.

Alternativ kann die Rotation des elektrostatischen Gleichfeldes mit einer Frequenz von 1 Hz bis 100 kHz, besonders bevorzugt von 20 Hz bis 65 kHz modulierbar ausgestaltet sein. Besonders bevorzugt ist die Rotation des elektrostatischen Gleichfeldes mit einer Frequenz von 1 kHz bis 100 kHz, ganz besonders bevorzugt von 20 kHz bis 65 kHz modulierbar. In einer besonderen Ausführung des erfindungsgemäßen Dialysegerätes ist die Rotation des elektrostatischen Gleichfeldes mit einer Frequenz von 1 Hz bis 100 Hz, bevorzugt von 20 Hz bis 65 Hz modulierbar. Durch die Rotation des elektrostatischen Gleichfeldes kann der Aufbau einer statischen Helmholtzdoppelschicht verhindert werden.

Das erfindungsgemäße Dialysegerät kann zusätzlich eine Regel- und/oder Steuereinheit aufweisen. Diese Regel- und/oder Steuereinheit kann derart ausgebildet sein, dass darüber Parameter des elektrostatischen Gleichfeldes und/oder des hochfrequenten elektromagnetischen Feldes regel- und/oder steuerbar sind. Dabei kann es sich um solche Parameter wie z. B. die Frequenz, die Feldstärke, die magnetische Flussdichte und/oder die Dauer des Feldes handeln. Dazu kann die Regel- und/oder Steuereinheit eine Eingabeeinheit, eine Recheneinheit und ggf. eine Speichereinheit umfassen über die Parameter des betreffenden Feldes durch einen Benutzer des Dialysegerätes regel- und/oder steuerbar sind. In einer bevorzugten Ausführungsform ist die Regel- und/oder Steuereinheit derart ausgebildet, dass sich darüber auch Parameter des Dialysekreislaufs und/oder des Blutkreislaufes, wie z. B. die Flussgeschwindigkeit des zu reinigenden Blutes und/oder der Dialysatflüssigkeit und/oder des Dialysates, durch einen Benutzer regeln und/oder steuern lassen.

Im Folgenden wird die Erfindung anhand von Ausführungsbeispielen näher erläutert.
Figuren:
- Figur 1: zeigt eine schematische Darstellung eines erfindungsgemäßen Dialysegerätes.
- Figur 2: zeigt die Urämie-Toxin-Menge (rel. Peakflächen) im Filtrat in An- und Abwesenheit eines HF-Feldes (OH-HPA=p-Hydroxyhippursäure; PAA=Phenylessigsäure; IDS=Indoxylsulfat).
- Figur 3: zeigt die Protein-Konzentration im Filtrat in An- und Abwesenheit eines HF-Feldes bei zwei baugleichen Modulen (kein signifikanter Unterschied)
- Figur 4: zeigt die Konzentration von Phenylessigsäure (PAA) im Retentat in Abhängigkeit der Frequenz des hochfrequenten elektromagnetischen (HF-) Feldes
- Figur 5: zeigt die Konzentration von Phenylessigsäure (PAA) im Retentat in Abhängigkeit von der Rotationsfrequenz des elektrostatichen (H-) Feldes
- Figur 6: zeigt die Konzentration von Phenylessigsäure (PAA) im Retentat in Abhängigkeit von der Feldstärke eines hochfrequenten elektromagnetischen (HF-) Feldes

### Beispiele:

### Beispiel 1: Beschreibung eines erfindungsgemäßen Dialysegerätes

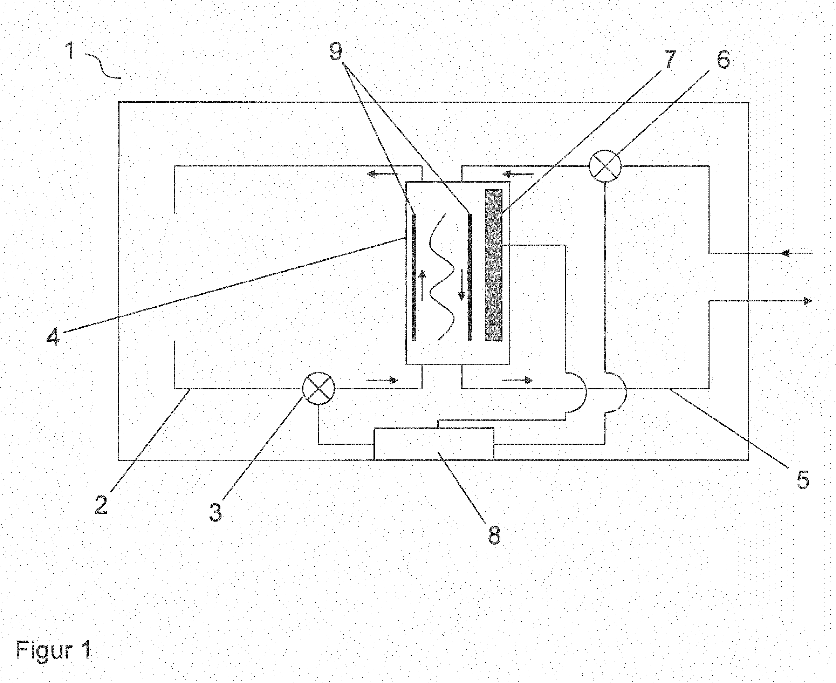

In Figur 1 ist ein erfindungsgemäßes Dialysegerät 1 schematisch dargestellt, welches sich zur Durchführung der erfindungsgemäßen Verwendung eignet. Das Dialysegerät 1 weist einen Dialysatkreislauf 2, einen Blutkreislauf 5 und einen Dialysator 4 auf, die derart miteinander verbunden vorliegen, dass im Dialysator 4 zu reinigendes Blut im Blutkreislauf 5 und Dialysat im Dialysatkreislauf 2 im Gegenstrom auf unterschiedlichen Seiten der semipermeablen Membran aneinander vorbeigeführt werden können, so dass ein Stoffaustausch zwischen Blut und Dialysat über die semipermeable Membran des Dialysators 4 ermöglicht ist. Für den gerichteten Transport von Blut im Blutkreislauf 5 kann eine Pumpe 6 vorgesehen sein. Für den gerichteten Transport von Dialysat im Dialysatkreislauf kann eine Dialysatpumpe 3 vorgesehen sein. Der Dialysator 4 kann beispielsweise als Kapillardialysator mit einer semipermeablen Membran mit einem Größenausschluss von 10.000 Da bis 25.000 Da ausgestaltet sein. Grundsätzlich kann der Aufbau des erfindungsgemäßen Dialysegerätes 1 aufgrund bekannter, herkömmlicher Dialysetechnologie erfolgen, wobei im Wesentlichen alle bekannten Dialysegeräte bzw. Dialysevorrichtungen als Basis verwendet werden können. Zusätzlich weist der Dialysator 4 Mittel 7 zur Erzeugung eines hochfrequenten elektromagnetischen Feldes sowie Mittel 9 zur Erzeugung eines elektrostatischen Gleichfeldes auf. Solche Mittel 7 können beispielsweise durch eine Hochfrequenzspule, eine Hochfrequenz-elektrode und/oder einen Hochfrequenzkondensator verkörpert sein. Die Mittel 9 können beispielsweise als elektrische Leiter oder Feldplatten ausgebildet sein, die auf gegenüberliegenden Seiten des Dialysators 4 angeordnet sind, so dass sich die Dialysemembran des Dialysators 4 zwischen den beiden Leitern oder Platten befindet. Das erfindungsgemäße Dialysegerät 1 kann zusätzlich eine Regel- und/oder Steuereinheit 8 aufweisen. Diese Regel- und/oder Steuereinheit 8 kann derart mit den Mitteln 7 und/oder den Mitteln 9 verbunden und ausgebildet sein, dass darüber Parameter der Mittel 7 zur Erzeugung eines hochfrequenten elektromagnetischen Feldes und/oder Mittel 9 zur Erzeugung eines elektrostatischen Gleichfeldes regel- und/oder steuerbar sind. Dabei kann es sich um solche Parameter wie z. B. die elektrische Frequenz, die elektrische Feldstärke, die magnetische Flussdichte und/oder die Dauer des betreffenden Feldes handeln. Dazu kann die Regel- und/oder Steuereinheit 8 eine Eingabeeinheit, eine Recheneinheit und eine Speichereinheit umfassen über die der Benutzer des Dialysegerätes 1 die Parameter des hochfrequenten elektromagnetischen Feldes und/oder Parameter des elektrostatischen Gleichfeldes regeln und/oder steuern kann. In einer bevorzugten Ausführungsform ist die Regel- und/oder Steuereinheit 8 derart ausgebildet, dass sich darüber auch Parameter des Dialysekreislaufs 2 und/oder des Blutkreislaufes 5, wie z. B. die Flussgeschwindigkeiten des zu reinigenden Blutes und/oder des Dialysates, durch einen Benutzer regeln und/oder steuern lassen.

### Beispiel 2 Nachweis des Effektes

In *in*-vitro-Versuchsreihen wurde der Einfluss hochfrequenter elektromagnetischer Felder auf den proteingebundenen Anteil der Urämietoxine untersucht. Hierzu wurde ein Dialysemodul erstellt, indem konventionelle Hämofiltrations-Kapillaren mit Hilfe von Silikon als Schlaufen in einen Spritzenaufnahmestutzen eingegossen wurden. In das betreffende Modul wurde eine wässerige Albumin-Lösung in Gegenwart der Urämie-Toxine Phenylessigsäure, p-Hydroxyhippursäure und Indoxylsulfat eingebracht. Mit Hilfe einer Spritzenpumpe wurde diese Lösung mit dem Dialysemodul für 10 min filtriert. Anschließend wurde durch Verwendung einer Hochfrequenz-Elektrode (HF-Elektrode) ein hochfrequentes elektromagnetisches Feld in der Lösung induziert. Das elektromagnetische Feld wird mittels einer Hochfrequenzspannungsquelle über einen Zeitraum von 10 Minuten von 1 bis 20 MHz in 1 MHz-Schritten inkrementiert. In den resultierenden Filtraten wurde die Konzentration der zuvor zum künstlichen Plasma gegebenen Urämie-Toxine Phenylessigsäure, p-Hydroxyhippursäure und Indoxylsulfat bestimmt. Durch Vergleich der Urämie-Toxin-Konzentrationen in den resultierenden Filtraten konnte der Effekt des HF-Feldes auf die Bindung zwischen Proteinen und Urämie-Toxinen evaluiert werden.

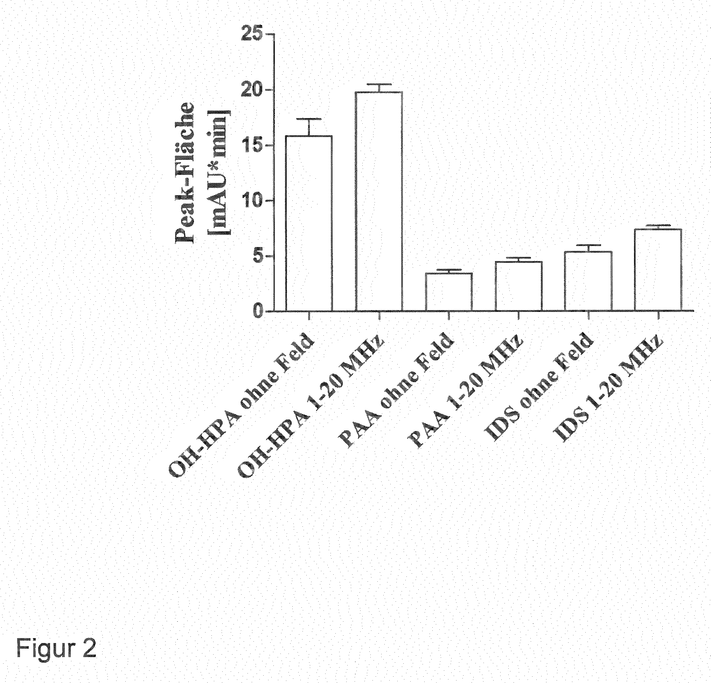

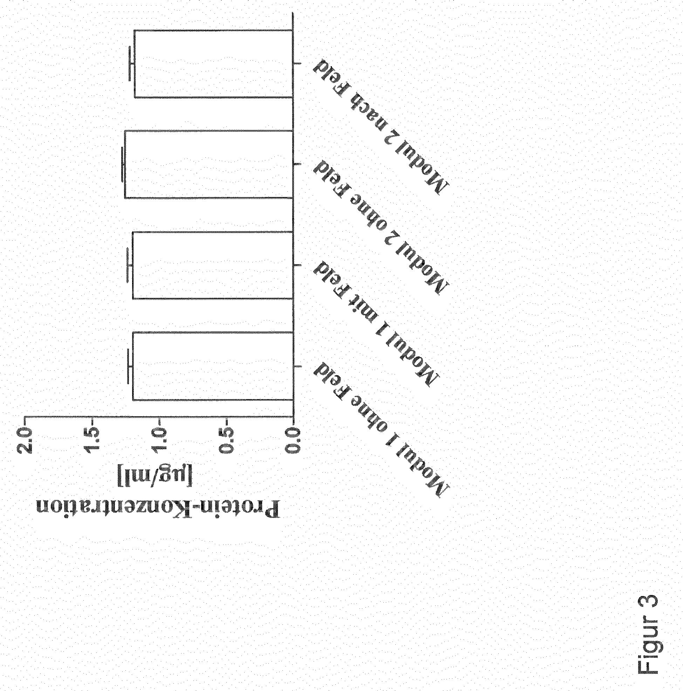

Die quantitative Bestimmung der Urämie-Toxin-Konzentration in den resultierenden Filtraten zeigte, dass hochfrequente elektromagnetische Felder die Filtrationsraten proteingebundener Urämie-Toxine signifikant erhöhen (Figur 2). Um zu überprüfen, ob hochfrequente elektromagnetische Felder die Dialysemembranen beschädigen, wurde die Proteinkonzentration im Filtrat mittels Proteinfärbung nach Bradford bestimmt. Die Ergebnisse zeigen, dass keine signifikanten Änderungen der Proteinkonzentration in Dialysemodulen ohne und unter Einfluss von hochfrequenten elektromagnetischen Feldern nachweisbar sind (Figur 3). Eine makroskopische Beschädigung der Membran ist aufgrund dieser Daten auszuschließen.

### Beispiel 3: Nachweis des Effektes in Abhängigkeit des HF-Feldes

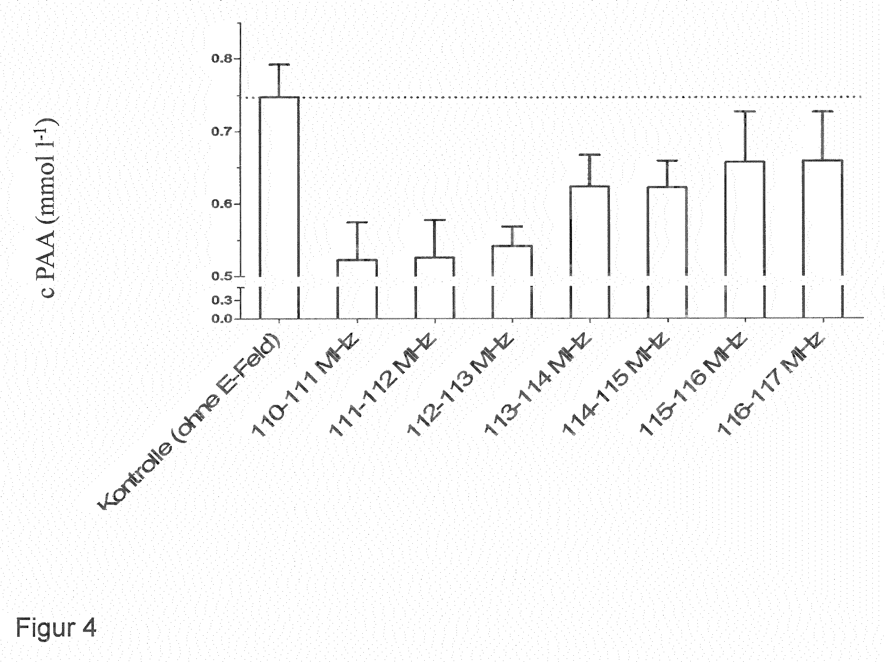

In weiterführenden Untersuchungen konnte im Besonderen der Frequenzbereich um 110-115 MHZ als effektiver Frequenzbereich zur Freisetzung proteingebundener Urämietoxine identifiziert werden. Der Versuchsaufbau gleicht dem des Beispiels 2, wobei andere Frequenzbereiche für das hochfrequente elektromagnetische (HF-) Feld eingesetzt wurden. In Figur 4 ist der Effekt der verwendeten Frequenzen auf die entsprechende Freisetzung und im Anschluss Abtrennung von Phenylessigsäure (PAA) dargestellt. Im Experiment wurde keine messbare Erwärmung des Blutplasmas beobachtet. Die hier gemessene Abtrennung der proteingebundenen Toxine beruht damit nicht auf einem thermischen Effekt.

Dabei hat sich gezeigt, dass sich die im Folgenden summarisch genannten Frequenzbereiche in besonderem Maße zur Abtrennung proteingebundener Urämietoxine eignen. Bei den betreffenden Frequenzbereichen handelt es sich um die Bereiche, bei denen der maximale Abtrenneffekt bestimmt worden ist. In den nicht-genannten Frequenzbereichen wurde teilweise eine im Vergleich zu Kontrolle vermehrte Abtrennung bestimmt, die jedoch geringer war, als in den unten genannten Frequenzbereichen.

| **Geeignete Frequenzen im HF-Feld (Stand 05.12.13)** | | | |
|---|---|---|---|
| **Frequenzen E-Feld** | **PAA** | **IDS** | **pCRS** |
| **80**-**120 MHz** | 110 | 110 | 110 |
| | 110-111 | 110-111 | 110-111 |
| | 111 | 111 | 111 |
| **120-170 MHz** | 140-141 | 140-141 | |
| | 148-149 | | 140-141 |
| | 160-161 | | 151-152 |

### Beispiel 4: Nachweis des Effektes in Abhängigkeit der Frequenz des H-Feldes

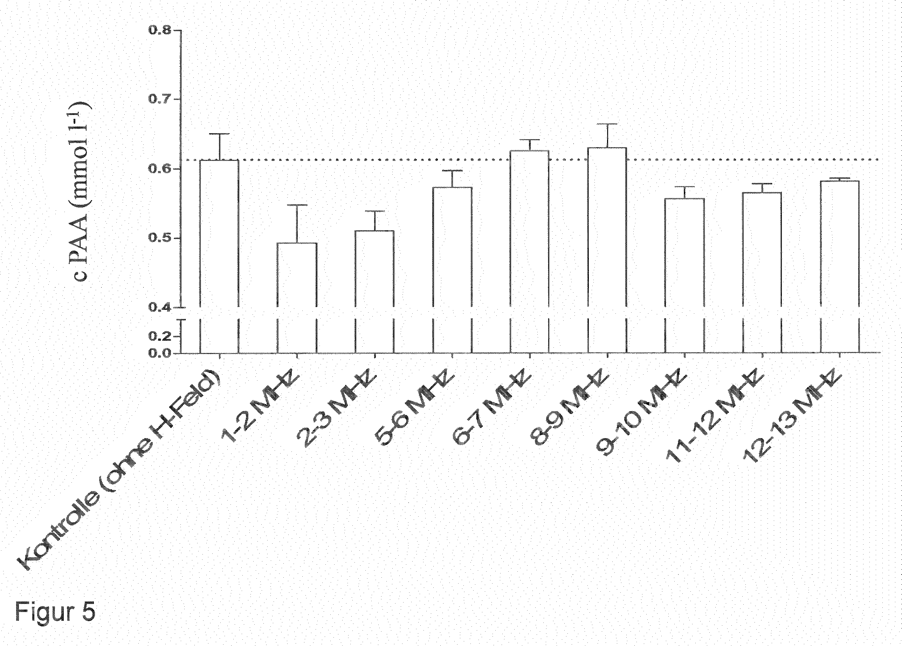

Der Versuchsaufbau gleicht im Wesentlichen dem des Beispiels 2, wobei anstatt des HF-Feldes nun ausgewählte Frequenzbereiche für das elektrostatische (H-) Feld geprüft wurden. Dabei konnte auch im Bereich des H-Feld eine vermehrte Freisetzung und damit Abtrennung der proteingebundener Urämietoxine bestimmt werden. Der Figur 5 ist zu entnehmen, dass der H-Feld-Bereich von 1-6MHz sowie der Bereich 9-13 MHZ besonders geeignet ist, proteingebundene Urämietoxine aus der Proteinbindung freizusetzen und in Folge dialytisch abzutrennen (gezeigt ist der Effekt auf Phenylessigsäure). Im Experiment wurde keine messbare Erwärmung des Blutplasmas beobachtet. Die hier gemessene Abtrennung der proteingebundenen Toxine beruht damit nicht auf einem thermischen Effekt.

### Beispiel 5: Effekt der Feldstärke

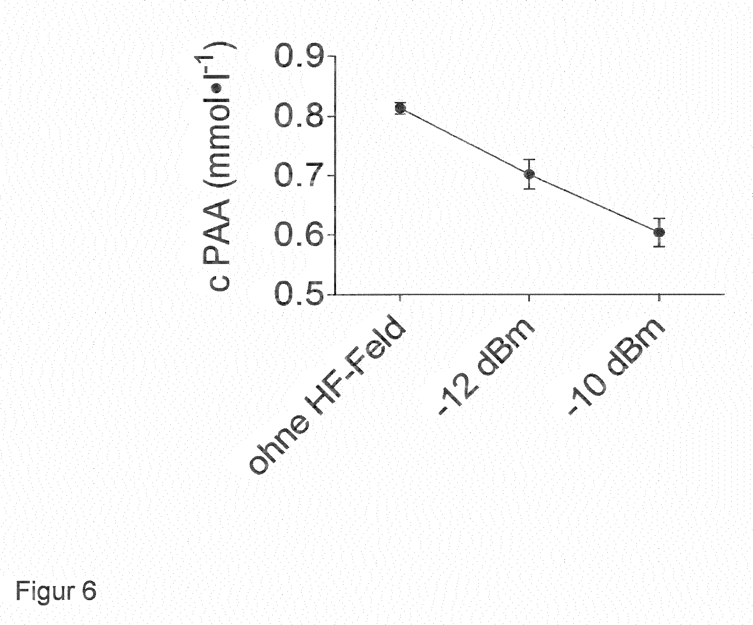

Neben der Frequenz des eingesetzten HF-Feldes ist auch dessen Feldstärke für die resultierende Freisetzung und Abtrennung relevant. Mit zunehmender Feldstärke werden vermehrt die betreffenden Urämietoxine aus der Proteinbindung freigesetzt und anschließend abtrennt. In Figur 6 ist dieser Effekt einer ansteigenden Feldstärke auf den Gehalt proteingebundener Urämietoxine im Retentat am Beispiel der Phenylessigsäure gezeigt. Im Experiment wurde keine messbare Erwärmung des Blutplasmas beobachtet. Die hier gemessene Abtrennung der proteingebundenen Toxine beruht damit nicht auf einem thermischen Effekt.

### Bezugszeichenliste:

- 1: Dialysegerät
- 2: Dialysatkreislauf
- 3: Dialysatpumpe
- 4: Dialysator
- 5: Blutkreislauf
- 6: Pumpe
- 7: Mittel zur Erzeugung eines hochfrequenten elektromagnetischen Feldes
- 8: Regel- und/oder Steuereinheit
- 9: Mittel zur Erzeugung eines elektrostatischen Gleichfeldes

## Patentansprüche

1. Dialysegerät (1) umfassend einen Dialysatkreislauf (2), einen Blutkreislauf (5) und einen Dialysator (4), wobei
das Dialysegerät Mittel (7) aufweist zur Erzeugung eines hochfrequenten elektromagnetischen Feldes sowie Mittel (9) zur Erzeugung eines elektrostatischen Gleichfeldes, wobei beide Mittel derart ausgeführt und angeordnet sind, dass zu reinigendes Blut während der Passage durch den Dialysator dem hochfrequenten elektromagnetischen Feld und dem elektrostatischen Gleichfeld aussetzbar ist,
wobei die Mittel zur Erzeugung eines elektrostatischen Gleichfeldes mindestens zwei elektrische Leiter aufweisen zwischen denen das elektrostatische Gleichfeld erzeugt wird, wobei die zwei elektrischen Leiter auf gegenüberliegenden Seiten des Dialysators angeordnet sind,
wobei die Mittel zur Erzeugung eines elektrostatischen Gleichfeldes mehr als zwei gegenüberliegende elektrische Leiter aufweisen,
**dadurch gekennzeichnet, dass**
die elektrischen Leiter derart um den Dialysator angeordnet sind, dass das elektrostatische Gleichfeld um die Achse entlang der Flussrichtung des zu reinigenden Blutes durch den Dialysator rotierbar ist.

2. Dialysegerät nach Anspruch 1, wobei die Mittel zur Erzeugung eines hochfrequenten elektromagnetischen Feldes und die Mittel zur Erzeugung eines elektrostatischen Gleichfeldes derart angeordnet sind, dass sich das hochfrequente, elektromagnetische Feld und das elektrostatische Gleichfeld ganz oder teilweise überlagern.

3. Dialysegerät nach Anspruch 1, wobei die Mittel zur Erzeugung eines hochfrequenten elektromagnetischen Feldes und die Mittel zur Erzeugung eines elektrostatischen Gleichfeldes derart angeordnet sind, dass das elektrostatische Gleichfeld dem hochfrequenten, elektromagnetischen Feld in Flussrichtung des zu reinigenden Blutes nachgeschaltet ist.

4. Dialysegerät nach einem der vorhergehenden Ansprüche, wobei die Mittel zur Erzeugung des elektrostatischen Gleichfeldes derart ausgebildet und angeordnet sind, dass das elektrostatische Gleichfeld nicht im Wesentlichen parallel zur Flussrichtung des zu reinigenden Blutes durch den Dialysator ausgerichtet ist.

5. Dialysegerät nach einem der vorhergehenden Ansprüche, wobei die Rotation des elektrostatischen Gleichfeldes mit einer Frequenz von 100 kHz bis 100 MHz, bevorzugt von 0,5 MHz bis 50 MHz, besonders bevorzugt von 1 MHz bis 25 MHz modulierbar ist.

6. Dialysegerät nach einem der vorhergehenden Ansprüche, wobei die elektrische Feldstärke des elektrostatischen Gleichfeldes ≤ 5000 V/m beträgt.

7. Dialysegerät nach einem der vorhergehenden Ansprüche, wobei die Mittel zur Erzeugung eines hochfrequenten elektromagnetischen Feldes eine Hochfrequenzspule, eine Hochfrequenzelektrode und/oder einen Hochfrequenzkondensator umfassen oder daraus bestehen.

8. Dialysegerät nach einem der vorhergehenden Ansprüche, wobei das Dialysegerät eine Regel- und/oder Steuereinheit umfasst, über die Parameter des elektrostatischen Gleichfeldes regel- und/oder steuerbar sind.

9. Dialysegerät nach einem der vorhergehenden Ansprüche, wobei das Dialysegerät derart ausgebildet ist, dass das zu reinigende Blut während der gesamten oder einem Teil der Passage durch den Dialysator dem hochfrequenten elektromagnetischen Feld aussetzbar ist.

10. Dialysegerät nach einem der vorhergehenden Ansprüche, wobei das Dialysegerät derart ausgebildet ist, dass das zu reinigende Blut während der gesamten oder einem Teil der Passage durch den Dialysator dem elektrostatischen Gleichfeld aussetzbar ist.

11. Dialysegerät nach einem der vorhergehenden Ansprüche, wobei die Mittel zur Erzeugung eines hochfrequenten elektromagnetischen Feldes mit einer Frequenz von 10 MHz bis 500 MHz, bevorzugt von 80 MHz bis 170 MHz, besonders bevorzugt von 100 MHz bis 120 MHz ausgestaltet sind.

12. Dialysegerät nach einem der vorhergehenden Ansprüche, wobei die Mittel zur Erzeugung eines hochfrequenten elektromagnetischen Feldes mit einer elektrischen Feldstärke von 1 bis 250 V/m ausgestaltet sind.

13. Dialysegerät nach einem der vorhergehenden Ansprüche, wobei die Mittel zur Erzeugung eines hochfrequenten elektromagnetischen Feldes und/oder die Mittel zur Erzeugung eines elektrostatischen Gleichfeldes integraler Bestandteil des Dialysators sind.

## Claims

1. A dialysis device (1) comprising
a dialysis circulation (2), a blood circulation (5)
and a dialysis machine (4),
wherein
the dialysis device features means (7) for generating a high-frequency electromagnetic field and means (9) for generating an electrostatic direct current field,
wherein both means are designed and arranged such that the blood to be purified can, during its passage through the dialysis machine, be exposed to the high-frequency electromagnetic field and the electrostatic direct current field,
wherein the means for generating an electrostatic direct current field feature at least two electrical conductors, between which the electrostatic direct current field is generated, wherein the two electrostatic conductors are arranged on opposite sides of the dialysis machine,
wherein the means for generating an electrostatic direct current field feature more than two opposite electrical conductors,
**characterized in that**
the electrical conductors are arranged about the dialysis machine in such a manner that the electrostatic direct current field can be rotated about the axis along the flow direction of the blood to be purified through the dialysis machine.

2. The dialysis device according to Claim 1, wherein the means for generating a high-frequency electromagnetic field and the means for generating an electrostatic direct current field are arranged such that the high-frequency electromagnetic field and the electrostatic direct current field are entirely or partially overlapping.

3. The dialysis device according to Claim 1, wherein the means for generating a high-frequency electromagnetic field and the means for generating an electrostatic direct current field are arranged such that the electrostatic direct current field is switched downstream of the high-frequency electromagnetic field in the direction of flow of the blood to be purified.

4. The dialysis device according to any one of the preceding claims, wherein the means for generating the electrostatic direct current field are designed and arranged such that the electrostatic direct current field is not essentially aligned parallel to the direction of flow of the blood to be purified through the dialysis machine.

5. The dialysis device according to any one of the preceding claims, wherein the rotation of the electrostatic direct current field can be modulated with a frequency of 100 kHz to 100 MHz, preferably from 0.5 MHz to 50 MHz, particularly preferred, from 1 MHz to 25 MHz.

6. The dialysis device according to any one of the preceding claims, wherein the electrical field strength of the electrostatic direct current field is ≤ 5000 V/m.

7. The dialysis device according to any one of the preceding claims, wherein the means for generating a high-frequency electromagnetic field comprise or consist of a high frequency coil, a high-frequency electrode and/or a high-frequency capacitor.

8. The dialysis device according to any one of the preceding claims, wherein the dialysis device comprises a regulating and/or control unit, via which parameters of the electrostatic direct current field can be regulated and/or controlled.

9. The dialysis device according to any one of the preceding claims, wherein the dialysis device is designed such that the blood to be purified, during the entire or part of its passage through the dialysis machine, can be exposed to the high-frequency electromagnetic field.

10. The dialysis device according to any one of the preceding claims, wherein the dialysis device is designed such that the blood to be purified, during the entire or part of its passage through the dialysis machine, can be exposed to the electrostatic direct current field.

11. The dialysis device according to any one of the preceding claims, wherein the means for generating a high-frequency electromagnetic field are equipped with a frequency of 10 MHz to 500 MHz, preferably from 80 MHz to 170 MHz, particularly preferred from 100 MHz to 120 MHz

12. The dialysis device according to any one of the preceding claims, wherein the means for generating a high-frequency electromagnetic field are equipped with an electrical field strength of 1 to 250 V/m.

13. The dialysis device according to any one of the preceding claims, wherein the means for generating a high-frequency electromagnetic field and/or the means for generating an electrostatic direct current field are an integral part of the dialysis machine.

## Revendications

1. Appareil de dialyse (1), comprenant
un circuit de dialysat (2), un circuit de sang (5) et
un dialyseur (4),
l'appareil de dialyse présentant (7) des moyens pour la génération d'un champ électromagnétique à haute fréquence ainsi que des moyens (9) pour la génération d'un champ électrostatique continu, les deux ensembles de moyens étant réalisés et disposés de telle sorte que du sang devant être purifié peut être soumis au champ électromagnétique à haute fréquence et au champ électrostatique continu pendant le passage par le dialyseur, les moyens pour la génération d'un champ électrostatique continu présentant au moins deux conducteurs électriques entre lesquels le champ électrostatique continu est généré,
les deux conducteurs électriques étant disposés sur des côtés opposés du dialyseur,
les moyens pour la génération d'un champ électrostatique continu présentant plus de deux conducteurs électriques opposés l'un à l'autre,
**caractérisé en ce que**
les conducteurs électriques sont disposés autour du dialyseur de telle sorte que le champ électrostatique continu peut être pivoté sur l'axe parallèle à la direction du flux du sang devant être nettoyé passant par le dialyseur.

2. Appareil de dialyse selon la revendication 1, les moyens pour la génération d'un champ électromagnétique à haute fréquence et les moyens pour la génération d'un champ électrostatique continu étant disposés de telle sorte que le champ électromagnétique à haute fréquence et le champ électrostatique continu se chevauchent totalement ou partiellement.

3. Appareil de dialyse selon la revendication 1, les moyens pour la génération d'un champ électromagnétique à haute fréquence et les moyens pour la génération d'un champ électrostatique continu étant disposés de telle sorte que le champ électrostatique continu est en aval du champ électromagnétique à haute fréquence dans la direction du flux de sang devant être nettoyé.

4. Appareil de dialyse selon l'une quelconque des revendications précédentes, les moyens pour la génération du champ électrostatique continu étant formés et disposés de telle sorte que le champ électrostatique continu n'est pas essentiellement orienté parallèlement à la direction du flux de sang devant être nettoyé passant par le dialyseur.

5. Appareil de dialyse selon l'une quelconque des revendications précédentes, la rotation du champ électrostatique continu étant modulable avec une fréquence de 100 kHz à 100 MHz, de préférence de 0,5 à MHz à 50 MHz, en particulier de préférence de 1 MHz à 25 MHz.

6. Appareil de dialyse selon l'une quelconque des revendications précédentes, l'intensité de champ électrique du champ électrostatique continu étant ≤ 5 000 V/m.

7. Appareil de dialyse selon l'une quelconque des revendications précédentes, les moyens pour la génération d'un champ électromagnétique à haute fréquence comprenant une bobine à haute fréquence, une électrode à haute fréquence et/ou un condensateur à haute fréquence, ou s'en composant.

8. Appareil de dialyse selon l'une quelconque des revendications précédentes, l'appareil de dialyse comprenant une unité de réglage ou de commande, par laquelle les paramètres du champ électrostatique continu sont réglables ou contrôlables.

9. Appareil de dialyse selon l'une quelconque des revendications précédentes, l'appareil de dialyse étant formé de telle sorte que le sang devant être nettoyé peut être soumis au champ électromagnétique à haute fréquence pendant la totalité ou une partie du passage par le dialyseur.

10. Appareil de dialyse selon l'une quelconque des revendications précédentes, l'appareil de dialyse étant formé de telle sorte que le sang devant être nettoyé peut être soumis au champ électrostatique continu pendant la totalité ou une partie du passage par le dialyseur.

11. Appareil de dialyse selon l'une quelconque des revendications précédentes, les moyens pour la génération d'un champ électromagnétique à haute fréquence étant conçus avec une fréquence de 10 MHz à 500 MHz, de préférence de 80 MHz à 170 MHz, en particulier de préférence de 100 MHz à 120 MHz.

12. Appareil de dialyse selon l'une quelconque des revendications précédentes, les moyens pour la génération d'un champ électromagnétique à haute fréquence étant conçus avec une force de champ électrique de 1 à 250 V/m.

13. Appareil de dialyse selon l'une quelconque des revendications précédentes, les moyens pour la génération d'un champ électromagnétique à haute fréquence et/ou les moyens pour la génération d'un champ électrostatique continu faisant partie intégrante du dialyseur.
